# EUROPEAN PATENT APPLICATION

(11) **EP 1 421 952 A1**
(43) Date of publication of application: **26.05.2004**
(21) Application number: 02796351.1
(22) Date of filing: 21.08.2002
(51) Int. Cl.: A61K 45/00, A61P 9/00, A61P 9/10, A61P 43/00, G01N 33/15, G01N 33/50

(54) **MYOCARDIAL CELL APOPTOSIS INHIBITORS**

(30) Priority: 22.08.2001 JP 2001000251
(71) Applicant: Yokota, Mitsuhiro, Nagoya-shi, Aichi 458-0812 (JP)
(72) Inventor: Yokota, Mitsuhiro, Nagoya-shi, Aichi 458-0812 (JP)
(74) Representative: VOSSIUS & PARTNER
(86) International application number: PCT/JP2002/008412
(87) International publication number: WO 2003/018058

(57) **Abstract**

The present invention utilizes a compound having a K_{ATP} channel opening effect and/or an NO donating effect as an active ingredient to provide an inhibitor of apoptosis in cardiac myocytes, a therapeutic agent for ischemic reperfusion injury, a therapeutic agent for myocardial infarction, a therapeutic agent for myocardiopathy and a therapeutic agent for myocarditis. The present invention also provides a method for screening a compound having at least one of the effects of inhibiting apoptosis in cardiac myocytes, inhibiting ischemic reperfusion injury, inhibiting myocardial infarction, inhibiting myocardiopathy and inhibiting myocarditis.

## Description

### TECHNICAL FIELD

The present invention relates to an inhibitor of apoptosis in cardiac myocytes, a therapeutic agent for ischemic reperfusion injury and a therapeutic agent for myocardial infarction, each of which comprises a compound having a K_{ATP} channel opening effect and/or an NO donating effect as an active ingredient; a method for inhibiting apoptosis in cardiac myocytes by using the compound; a method for screening a compound having at least one of the effects of inhibiting apoptosis in cardiac myocytes, inhibiting ischemic reperfusion injury and inhibiting myocardial infarction; and a kit which comprises a compound having a K_{ATP} channel opening effect and/or an NO donating effect in an amount effective for inhibiting apoptosis in cardiac myocytes, treating ischemic reperfusion injury or treating myocardial infarction, and instructions for use.

### BACKGROUND ART

As a consequence of ischemic heart diseases (e.g., myocardial infarction, angina pectoris), hypoxia caused by reduction or interruption of coronary blood flow inhibits energy production in cardiac myocytes, leading to decreased heart function as well as irreversible cell damage in cardiac myocytes. For this reason, once ischemic heart diseases have occurred, blood flow should be rapidly recovered. However, it is well known that when oxygen supply is restarted by reperfusion after ischemia in order to recover coronary blood flow, such treatment causes further exacerbation of myocardial damage such as myocardial necrosis or stunned myocardium. In particular, the increase in infarct size after reperfusion is called ischemic reperfusion injury; there has been a need to develop a method or agent for inhibiting ischemic reperfusion injury.

Ischemic reperfusion injury is induced by various factors. In addition to necrosis, the induction of apoptosis may be one of the factors in cardiac myocytes. Until now, it has been suggested that ischemic preconditioning (IP), which is known to be involved in the inhibition of ischemic reperfusion injury, prevents neutrophil accumulation and represses BAX expression, thereby reducing the incidence of apoptosis in cardiac myocytes.

On the other hand, nicorandil (NCR) is shown to have not only a nitrate-like NO donating effect (NO donor effect), but also a K_{ATP} channel opening effect. This compound is also confirmed to decrease infarct size in animal models of ischemic reperfusion (Sanada Y. et al., American Journal of Physiology, 280(1), 256-263 (2001); Menasche P. et al., Journal of Cardiovascular Surgery, 110(6), 1606-1613 (1995)). Apoptosis induced by oxidative stress in cardiac myocytes is known to cause an increase in the expression level of p53, but no change in the expression levels of Bax and Bcl-2. Apoptosis stimulated by hydrogen peroxide (H₂O₂) is known to increase the level of Bad protein and induce the translocation of Bax and Bad from cytosol to mitochondria, which in turn causes cytochrome c release, CPP32 activation and poly (ADP-ribose) polymerase cleavage. However, it is also suggested that there are other mechanisms of inducing apoptosis(Harsdorf R. et al., Circulation, 99, 2934-2941 (1999)).

However, since ischemic reperfusion injury is caused by various factors, as stated above, it has been still unclear as to which mechanism is involved in inducing the ability of nicorandil to decrease infarct size. It has also been unclear whether nicorandil directly inhibits apoptosis in cardiac myocytes.

Since coronary blood flow is restarted under cell membrane damage of cardiac myocytes, it is believed to cause a rapid elevation in the intracellular level of calcium involved in signal transduction and impose a severe burden on the cells, thereby causing ischemic reperfusion injury. Recently, it is also believed that reperfusion injury is further amplified through the generation of free radicals such as active oxygen during reperfusion.

### DISCLOSURE OF THE INVENTION

The object of the present invention is to provide an inhibitor of apoptosis in cardiac myocytes, a therapeutic agent for ischemic reperfusion injury, a therapeutic agent for myocardial infarction, a therapeutic agent for myocardiopathy, and a therapeutic agent for myocarditis, as well as a method for screening a compound having at least one of the effects of inhibiting apoptosis in cardiac myocytes, inhibiting ischemic reperfusion injury, inhibiting myocardial infarction, inhibiting myocardiopathy and inhibiting myocarditis.

As a result of extensive and intensive efforts made to study the effect of nicorandil on the above-mentioned intracellular calcium overload and oxidative stress related to apoptosis in cardiac myocytes during ischemic reperfusion, the inventors of the present invention have found that nicorandil allows direct inhibition of apoptosis in cardiac myocytes resulting from intracellular Ca overload caused by calcium ionophore or oxidative stress caused by hydrogen peroxide. The inventors of the present invention have further clarified that the anti-apoptotic effect of nicorandil in cardiac myocytes results from both a K_{ATP} channel opening effect and an NO donating effect, and that PKC activation enhances Bad phosphorylation and hence inhibits apoptosis.

Namely, the present invention provides an inhibitor of apoptosis in cardiac myocytes, which comprises a compound having a K_{ATP} channel opening effect and/or an NO donating effect as an active ingredient.

The present invention also provides a therapeutic agent for ischemic reperfusion injury and/or a therapeutic agent for myocardial infarction and/or a therapeutic agent for myocardiopathy and/or a therapeutic agent for myocarditis, each of which comprises a compound having a K_{ATP} channel opening effect and/or an NO donating effect as an active ingredient.

Further, the present invention provides a therapeutic agent for ischemic reperfusion injury and/or a therapeutic agent for myocardial infarction and/or a therapeutic agent for myocardiopathy and/or a therapeutic agent for myocarditis, each of which comprises a compound having an NO donating effect as an active ingredient, wherein said therapeutic agent is used in combination with an agent having a K_{ATP} channel opening effect.

Furthermore, the present invention provides a therapeutic agent for ischemic reperfusion injury and/or a therapeutic agent for myocardial infarction and/or a therapeutic agent for myocardiopathy and/or a therapeutic agent for myocarditis, each of which comprises a compound having a K_{ATP} channel opening effect as an active ingredient, wherein said therapeutic agent is used in combination with an agent having an NO donating effect.

Furthermore, the present invention provides a method for inhibiting apoptosis in cardiac myocytes, which comprises exposing cardiac myocytes to a compound having a K_{ATP} channel opening effect and/or an NO donating effect in an amount sufficient to increase the level of phosphorylated Bad in the cardiac myocytes.

Further, the present invention provides a method for screening a compound having at least one of the effects of inhibiting apoptosis in cardiac myocytes, inhibiting ischemic reperfusion injury, inhibiting myocardial infarction, inhibiting myocardiopathy and inhibiting myocarditis, which comprises the following steps:
(1) exposing cells to a test compound; and
(2) determining the level of phosphorylated Bad protein present in the cells.

Further, the present invention provides an inhibitor of apoptosis in cardiac myocytes and/or a therapeutic agent for ischemic reperfusion injury and/or a therapeutic agent for myocardial infarction and/or a therapeutic agent for myocardiopathy and/or a therapeutic agent for myocarditis, each of which comprises a compound obtained by the above screening method as an active ingredient.

Further, the present invention provides a kit for inhibiting apoptosis in cardiac myocytes, which comprises a compound having a K_{ATP} channel opening effect and/or an NO donating effect in an amount effective for inhibiting apoptosis in cardiac myocytes, and instructions for use.

Further, the present invention provides a kit for treating ischemic reperfusion injury and/or treating myocardial infarction and/or treating myocardiopathy and/or treating myocarditis, which comprises a compound having a K_{ATP} channel opening effect and/or an NO donating effect in an amount effective for treating ischemic reperfusion injury and/or treating myocardial infarction and/or treating myocardiopathy and/or treating myocarditis, and instructions for use.

Further, the present invention provides a kit for treating ischemic reperfusion injury and/or treating myocardial infarction and/or treating myocardiopathy and/or treating myocarditis, which comprises a compound having an NO donating effect in an amount effective for treating ischemic reperfusion injury and/or treating myocardial infarction and/or treating myocardiopathy and/or treating myocarditis, and instructions for use, wherein said kit is used in combination with an agent having a K_{ATP} channel opening effect.

Further, the present invention provides a kit for treating ischemic reperfusion injury and/or treating myocardial infarction and/or treating myocardiopathy and/or treating myocarditis, which comprises a compound having a K_{ATP} channel opening effect in an amount effective for treating ischemic reperfusion injury and/or treating myocardial infarction and/or treating myocardiopathy and/or treating myocarditis, and instructions for use, wherein said kit is used in combination with an agent having an NO donating effect.

### BRIEF DESCRIPTION OF DRAWINGS

Figure 1 is a graph showing the results of an analysis where the cell culture of cardiac myocytes prepared as described in the examples was measured for the percentage of apoptotic cells by flow cytometry in the presence of H₂O₂, nicorandil, or nicorandil + H₂O₂.

Figure 2 is a graph showing the results of an analysis where the cell culture of cardiac myocytes prepared as described in the examples was measured for the percentage of apoptotic cells by flow cytometry in the presence of H₂O₂, nicorandil + H₂O₂, or 5-hydroxydecanoate + nicorandil + H₂O₂.

Figure 3 is a graph showing the results of an analysis where the cell culture of cardiac myocytes prepared as described in the examples was measured for the percentage of apoptotic cells by flow cytometry in the presence of H₂O₂, nicorandil, 5-hydroxydecanoate and glibenclamide, alone or in combination, at various concentrations.

Figure 4 is a graph showing the results of an analysis where the cell culture of cardiac myocytes prepared as described in the examples was measured for the percentage of apoptotic cells by flow cytometry in the presence of calcium ionophore A23187, ZVAD, ZVAD + calcium ionophore A23187, nicorandil + calcium ionophore A23187, or 5-hydroxydecanoate + nicorandil + calcium ionophore A23187.

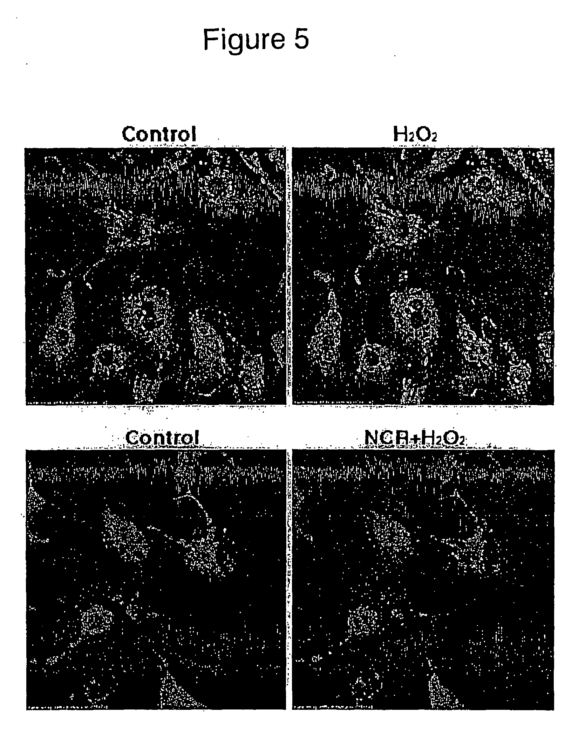

Figure 5 is a photograph showing fluorescence views of cells untreated (upper) and treated (lower) with nicorandil in the absence (left) and presence (right) of H₂O₂.

### BEST MODE FOR CARRYING OUT THE INVENTION

Cardiac myocytes refer to cells constituting the heart, particularly the heart wall, including ventricular and atrial cardiac myocytes. The heart wall is composed of three layers, endocardium, myocardium and epicardium. Cardiac myocytes as used herein will be derived from any of endocardium, myocardium and epicardium. Depending on the severity of infraction, the area of myocardial infarction can be localized within one layer or spread over multiple layers. Thus, the agent of the present invention is capable of inhibiting apoptosis in cardiac myocytes, ischemic reperfusion injury or myocardial infarction, in which such cardiac myocytes are involved.

Apoptosis is a form of cell death different from necrosis, which was proposed in 1972 by UK researchers H. Kerr and A. H. Wylle. Apoptosis is believed to occur through morphological processes of nuclear compaction, cellular shrinkage, cytosolic vacuolization and cell surface smoothing, widening of intercellular spaces, cell release from surrounding areas, cell fragmentation (apoptotic bodies), and phagocytosis by macrophages or the like. Apoptosis is biochemically characterized by activation of DNase and nucleosome-by-nucleosome fragmentation of DNA (180 bases). Thus, test cells can be identified to undergo apoptosis if a DNA ladder is analytically (e.g., electrophoretically) observed for the fragmented DNA extracted from the cells. In contrast, necrosis is mediated by processes of cell membrane/mitochondrial damage, cell swelling, energy production failure, intracellular osmotic dysregulation, and cytolysis. Thus, no clear DNA ladder can be confirmed for inecrotic cells. Moreover, necrosis causes inflammation around inecrotic cells by the action of enzymes such as protease released from the inecrotic cells, whereas apoptosis causes no inflammation around apoptotic cells.

The K_{ATP} channel is also called the ATP-sensitive K channel, which is a kind of ion channel for potassium transport and has the characteristic of being inhibited by intracellular ATP (Noma A., Nature, 305, 147-148 (1983)). The K_{ATP} channel is known to be expressed in many cells including nerve cells, pancreatic β-cells and smooth muscle cells. There are already many known compounds having a K_{ATP} channel opening effect, examples of which include nicorandil, cromakalim, lemakarim, pinacidil, aprikalim, minoxidil and diazoxide (Satoh Y., Folia Pharmacol. Japon, 100, 219-227 (1992)).

The compound having a K_{ATP} channel opening effect as used herein may be a compound that activates K_{ATP} channels expressed at least in cardiac myocytes, preferably a compound that specifically activates K_{ATP} channels expressed in cardiac myocytes. As used herein, a K_{ATP} channel opening effect is synonymous with a K_{ATP} channel activating effect; these effects both refer to the effect of inducing potassium ion transport through K_{ATP} channels. To determine the K_{ATP} channel opening effect, the cell membrane potential may be measured by the patch clamp technique using microelectrodes. At least, compounds having the same or a higher K_{ATP} channel opening activity than nicorandil can be used in the present invention.

There are already many known compounds allowing inhibition of K_{ATP} channels, examples of which include glibenclamide, tolbutamide and 4-aminopyridine. When appropriately combined, these compounds can be used to identify K_{ATP} channels per se and determine the selectivity of a compound having a K_{ATP} channel opening effect. To select a compound that specifically opens K_{ATP} channels expressed in cardiac myocytes, cardiac myocytes and other cells (e.g., nerve cells, pancreatic β-cells) may be provided and exposed to a test compound to measure the cell membrane potential for each cell.

The term "NO donating effect" refers to the effect of releasing NO (nitric oxide) in the body, which is also called the NO donor effect. Thus, a compound having an NO donating effect refers to a compound capable of serving as an NO source and releasing NO in the body. The phrase "in the body" means "in the blood" or "in cells." This phrase also covers intercellular spaces or extracellular matrixes.

A compound having an NO donating effect may or may not be naturally occurring in the body. Some mechanisms are known for NO release from a compound having an NO donating effect, which are divided into two processes: a chemical process for NO release through chemical decomposition and an enzymatic process for NO release. Examples of such a compound include sodium nitroprusside, hydroxylamine, acidified nitrates, molsidomine and sydonominine, as well as organic nitrites (R-ONO), organic nitrates (R-ONO₂) exemplified by nitroglycerin and nicorandil, and s-nitrosothiols (R-S-NO) (Bauer J.A. et al., Advances in Pharmacology, 34, 361-381 (1995)). Any compound falls within the scope of the compound having an NO donating effect as used herein, as long as it has the ability to release NO in the body.

There are various techniques known for measuring the NO donating effect, which may be selected as appropriate for use. Examples of such techniques include a technique based on the degree of vascular smooth muscle relaxation, color development using a Griess' reagent, a fluorescence technique using a DAN reagent (2,3-diaminonaphthalene), a chemoluminescence technique using luminol, an electron spin resonance technique using DBNBS (2,5-dibromo-4-nitrobenzene), spectroscopy based on metroglobulin, an electrical technique using porphyrin polymers, and NADPH-diaphorase staining using NBT (nitroblue tetrazolium). Agents having an NO donor activity, which can be used in the present invention, may have at least the same or a higher activity than nicorandil, as measured by any of the above techniques.

There is no particular limitation on the compound of the present invention and a test compound. Examples include synthetic organic compounds, peptides, non-peptide compounds, naturally occurring compounds, cell extracts, cell culture supernatants, microbial fermentation products, marine organism extracts, plant extracts, and purified or crude proteins. Preferred are synthetic organic compounds, and more preferred are synthetic low-molecular-weight compounds having a molecular weight of 2000 or less, preferably 1000 or less.

The term "calcium overload" refers to a state where the intracellular calcium level is elevated beyond the normal range. Calcium overload is induced in various diseases such as ischemic reperfusion injury, myocardial infarction, myocardiopathy and myocarditis. For *in vitro* elevation of intracellular calcium levels, a calcium ionophore such as A23187 may be used. The term "oxidative stress" refers to causing cellular dysfunction through protein oxidation or cleavage by free radicals such as active oxygen generated in the body, or refers to a process for causing cellular dysfunction. For *in vitro* induction of oxidative stress, a hydrogen peroxide solution may be added to a cell culture. Examples of diseases found to induce oxidative stress include ischemic reperfusion injury, myocardial infarction, myocardiopathy and myocarditis. Thus, both calcium overload and oxidative stress contribute to damage induction in cells during ischemic reperfusion injury, myocardial infarction, myocardiopathy and myocarditis.

Nicorandil, which is a common name for N-(2-hydroxyethyl)nicotinamide nitrate ester, is a compound commercially available as a therapeutic agent for angina pectoris. When used in the present invention, it can be used in the form of commercially available tablets or injectable formulations without further modification. Alternatively, it is also possible to use nicorandil prepared as described in, e.g., JP 52-122373 A.

The terms "inhibitor", "therapeutic agent" and "agent" each refer to a pharmaceutical composition capable of being administered as a medicament to a human body and comprising at least one active ingredient. In general, a pharmaceutical composition comprises pharmaceutically acceptable carriers or vehicles, in addition to an active ingredient(s), and is formulated in a known pharmaceutical manner. Examples of dosage forms include tablets, capsules and powders for oral administration and injections for parenteral administration. Examples of pharmaceutically acceptable carriers or vehicles include surfactants, stabilizers, emulsifiers, vegetable oils, excipients, preservatives, binders, lubricants, bulking agents and isotonizing agents, which may be used in combination as appropriate.

Examples of additives, which can be incorporated into tablets and capsules, include binders such as gelatin, corn starch, gum tragacanth or gum arabic; excipients such as crystalline cellulose; swelling agents such as corn starch, gelatin or alginic acid; lubricants such as magnesium stearate; sweetening agents such as sucrose, lactose or saccharin; and flavoring agents such as peppermint, *Gaultheria adenothrix* oil or cherry. In a case where the unit dosage form is a capsule, it may further contain liquid carriers such as fats and oils, in addition to materials of the above type.

Sterile compositions for injection may be formulated according to general pharmaceutical methods by using a vehicle such as distilled water for injection. Examples of an aqueous vehicle for injection include physiological saline and isotonic solutions containing glucose and/or other auxiliaries (e.g., D-sorbitol, D-mannose, D-mannitol, sodium chloride), which may be used in combination with appropriate solubilizers such as alcohols (e.g., ethanol) and nonionic surfactants (e.g., Polysorbate 80™, HCO-50).

Examples of an oil vehicle include sesame oil and soy bean oil, which may be used in combination with solubilizers such as benzyl benzoate and benzyl alcohol. Moreover, these vehicles may be blended with buffers (e.g., phosphate buffer, sodium acetate buffer), soothing agents (e.g., procaine hydrochloride), stabilizers (e.g., benzyl alcohol, phenol), antioxidants, etc. The prepared solutions for injection are usually filled into appropriate ampules or may be filled into syringes for direct use.

The kit of the present invention comprises an inhibitor, a therapeutic agent or an agent, each of which is formulated in a known pharmaceutical manner and comprises a compound having a K_{ATP} channel opening effect and/or an NO donating effect as an active ingredient in combination with pharmaceutically acceptable carriers or vehicles, as well as instructions for use.

Administration to a patient may be accomplished in a manner known to those skilled in the art, for example, by intraarterial, intravenous or subcutaneous injection or via the intranasal, transbronchial, intramuscular, percutaneous or oral route. Although the dose will vary depending on the weight and age of a patient, the severity of injury or symptoms, the intended route of administration and so on, those skilled in the art may select a suitable dose as appropriate. For example, when administered orally, the single dose may be 1 mg to 1000 mg, preferably 10 mg to 500 mg, and more preferably 50 mg to 300 mg.

Bad protein is a member of the Bcl-2 family and also believed to be one of the pro-apoptotic members. In general, the Bcl-2 family has all or part of Bcl-2 homology regions (BH1-4) and consists of anti-apoptotic and pro-apoptotic proteins. Anti-apoptotic proteins include Bcl-2, Bcl-XL, Bcl-W and MCL-1, whereas pro-apoptotic proteins include Bax, Bak, Bok/MTD and Bcl-Xs.

It is suggested that Bad protein has only the BH3 domain among the Bcl-2 homology regions and binds to anti-apoptotic Bcl-XL or the like, thereby inhibiting the anti-apoptotic action of Bcl-XL. It is also found that Bad protein undergoes phosphorylation on Ser residues at positions 112 and 136, which in turn controls the activity of Bad protein (Korsmeyer S.J. et al., Cold Spring Harbor Symposia on Quantitative Biology, 64, 343-350 (1999); Harsdorf R. et al., Circulation, 99, 2934-2941 (1999)).

To screen a compound that stimulates phosphorylation of Bad protein, cells (preferably, cardiac myocytes) may be exposed to a test compound to determine the phosphorylation level of Bad protein present in the cells. Cell exposure to a test compound may be accomplished by adding a solution of the test compound to a cell culture. A medium used to dissolve a test compound may be selected as appropriate for the nature of the test compound. For example, physiological saline or cell culture medium may be used for a test compound that is highly soluble in water. In contrast, an organic solvent such as ethanol, DMSO or DMF may be used for a test compound that is less soluble in water. To subtract the effect of an organic solvent, it is preferable to use the organic solvent alone as a control in the absence of a test compound.

To determine the level of phosphorylated Bad protein in the cells exposed to a test compound, intracellular proteins are first extracted from the cells. Techniques for extracting intracellular proteins may be appropriately selected by those skilled in the art. If necessary, cells which are not exposed to the test compound may be provided as a control to compare the level of phosphorylated Bad protein in the presence and absence of the test compound.

For qualitative or quantitative measurement of Bad protein, a commercially available anti-Bad protein antibody may be used. An antibody specific to phosphorylated Bad protein can also be purchased as a commercially available reagent. When an antibody that recognizes both phosphorylated and non-phosphorylatd Bad protein is used in combination with an antibody that specifically recognizes phosphorylated Bad protein, such a combination allows qualitative or quantitative measurement of phosphorylated Bad protein. For example, such an anti-Bad protein antibody may be directly labeled with an enzyme such as alkaline phosphatase, and the amount of the enzyme bound to Bad protein may be measured in various manners. In general, it is preferably accomplished by colorimetrically determining the enzyme-catalyzed conversion of a substrate. Detection may be accomplished by either dot blotting or ELISA. Alternatively, test samples may be electrophoresed and transferred to PVDF membranes to measure the amount present on the membranes. After reaction with an anti-Bad protein antibody, test samples may further be reacted with an enzyme-labeled secondary antibody against the anti-Bad antibody. For secondary antibody labeling, an enzyme such as alkaline phosphatase may also be used as in the case of the primary antibody.

### EXAMPLES

The present invention will be further described in the following Examples, which are not intended to limit the scope of the invention.

### Experimental materials and methods

### (1) Preparation of cardiac myocytes

Cultured neonatal rat ventricular myocytes (NRVMs) were provided as cardiac myocytes used in the experiments. More specifically, hearts were extracted from 0- to 1-day-old neonatal rats (Wister, pregnant rats were purchased from Chubu Shizai). The ventricles of each heart were then treated with collagenase Type 2 (Worthington) and pancreatin (Sigma) to enzymatically isolate cardiac myocytes (collagenase 0.5 mg/ml, pancreatin 0.6 mg/ml, 4 treatments), followed by primary culture. After the enzymatically collected cells were seeded in plates and allowed to stand for 30 minutes, non-adhesive cells were collected to remove fibroblasts. The resulting cardiac myocytes were seeded at a density of 1 × 10⁵ cells/cm² on 6-well plates or 75cm² flasks pre-coated with 1% gelatin and then cultured at 37°C under 5% CO₂ for 48 hours in a medium consisting of DMEM/Medium 199 (4:1), 10% fetal bovine serum (FBS, Sigma), 0.1 mM bromodeoxyuridine (BrdU, Roche), penicillin (50 units/ml, GIBCO) and streptomycin (50 µg/ml, GIBCO). Culturing was carried out in a CO₂ incubator under conditions of 37°C. The medium was then replaced with a serum-free medium which had been prepared from the same ingredients other than FBS and supplemented with insulin-transferrin-selenium (ITS, GIBCO). These cells were provided for experiments 24 hours after replacement with the serum-free medium. After immunocytostaining with a cardiac myocytespecific antibody (α-actinin antibody), the percentage of cardiac myocytes was measured by microscopy to confirm that 90% or more of the cells were cardiac myocytes at that stage.

### (2) Flow cytometry analysis

Flow cytometry analysis was performed using a Coulter EPICSXL and a software package specific for this purpose. After collection of floating cardiac myocytes from the cell culture (1 × 10⁶ cells/well), adhered cardiac myocytes were trypsinated and collected in the same tube. After centrifugation, the pellet was washed with PBS, supplemented with 70% EtOH, and then allowed to stand at room temperature for 20 minutes or more to fix the cells. After fixation, the cells were washed with PBS, re-suspended in 1 ml PBS and then reacted with 50 µL RNase (1 mg/ml, Sigma) at 37°C for 40 minutes. After the cells were allowed to stand at room temperature (if necessary), they were washed with PBS and suspended in about 250 µL PBS to prepare a cell suspension. Propidium iodide (250 µL, 25 µg/ml, Sigma) was added to and suspended well in the cell suspension, which was then allowed to stand at room temperature for 20 minutes or more and analyzed by flow cytometry. Cells showing a Sub-G1 peak in nuclear staining with propidium iodide were counted as apoptotic cells to calculate the percentage of cells showing a Sub-G1 peak relative to the total cell count measured (1 × 10⁴ cells). The calculated percentage was defined as the percentage of apoptotic cells.

### (3) Ladder assay

After floating and adhered cardiac myocytes were collected as stated above in the same tube, 370 µL lysis buffer (10 mM Tris-HCL, pH 8.0, 100 mM NaCl, 25 mM EDTA and 0.5% SDS) was added to collect total DNA. More specifically, the cell lysate was transferred to an Eppendorf tube and treated with proteinase K (GIBCO) at a final concentration of 1 mg/ml. The cell lysate was digested overnight at 37°C and then cooled on ice. NaCl was added at a final concentration of 1.3 M and the resulting precipitates were removed by centrifugation. The supernatant was extracted with phenol/chloroform and precipitated in EtOH to separate genomic DNA. The DNA was washed with 70% EtOH, re-suspended in TE (pH 8.0) and then digested with RNase (final concentration: 20 µg/ml) at 37°C for 30 to 60 minutes. Each lane was loaded with 10 pg DNA and electrophoresed on a 2% agarose gel. The gel was stained with ethidium bromide and then visualized under UV irradiation. Cells in which ladder formation was observed were defined as fragmented cells.

### (4) Immunocytochemistry

Cells were fixed with 4% paraformaldehyde and then treated with PBS containing 0.2% Triton X-100 and 2% normal goat serum for 30 minutes, followed by incubation with a mouse monoclonal α-actinin antibody (1 µg/ml, Sigma) and a mouse monoclonal antibody against native cytochrome C (1:500, Pharmingen) at room temperature for 1 hour. A FITC-conjugated goat anti-mouse IgG (1:100, MBL) was used as a secondary antibody. The nuclei were stained with a DNA-binding dye DAPI (Sigma).

### (5) Western Blot Analysis

Both floating and adhered cardiac myocytes were collected and lysed at 4°C for 1 hour in a lysis buffer consisting of 20 mM Tris-HCl (pH 7.5), 2 mM EDTA, 3 mM EGTA, 2 mM DTT, 250 mM sucrose, 1% Triton X-100, 10 µg/ml aprotinin, 10 µM leupeptin and 0.1 mM PMSF. The resulting cell lysate was measured for its protein level by the Bradford method, solubilized in 2X sample buffer and then boiled at 95°C for 3 minutes. After each lane was loaded with 50 µg sample and electrophoresed on a 12.5% SDS-polyacrylamide gel, the gel was transferred to a PVDF membrane. The blots were blocked overnight in 3% BSA/0.1% Tween-20 in PBS and then detected with the following primary antibodies: rabbit polyclonal Bcl-2 antibody (Pharmingen), rabbit polyclonal Bax antibody (Pharmingen), mouse monoclonal p53 antibody (Calbiochem), mouse monoclonal poly(ADP-ribose) polymerase (PARP) antibody (Clontech) and rabbit polyclonal CPP32 antibody (Santa Cruz). Bad was immunoprecipitated with a mouse monoclonal Bad antibody and collected by protein G-sepharose. After electrophoresis on a 15% SDS-polyacrylamide gel, the gel was transferred to a PVDF membrane and probed with a mouse monoclonal Phospho-Bad (phosphorylated Bad) (Ser-136) antibody (New England Biolabs).

### (6) Cellular fractionation

Cardiac myocytes were washed twice with ice-cold PBS and collected by centrifugation at 4°C at 200 × g for 10 minutes. The pellet was re-suspended in 200 µL extraction buffer (20 mM HEPES (pH 7.4), 220 mM mannitol, 68 mM sucrose, 50 mM KCl, 5 mM EGTA, 1 mM MgCl₂, 1 mM DTT, 10 µg/ml aprotinin, 10 µM leupeptin, 0.1 mM PMSF). The cells were homogenized with 20 strokes in a Dounce-type homogenizer. The cell homogenate was transferred to an Eppendorf tube and centrifuged at 4°C at 14000 × g for 30 seconds to precipitate the nuclei. The supernatant was transferred to an ultracentrifuge tube and centrifuged at 4°C at 200000 × g for 30 minutes. The supernatant (soluble proteins) and pellet (mitochondria, microsomes, and insoluble proteins) were measured for their protein level by the Bradford method. After the supernatant and pellet were solubilized with SDS, each lane was loaded with 2 µg sample and electrophoresed on a 12.5% SDS-polyacrylamide gel. The gel was transferred to a PVDF membrane. The Blots were blocked overnight in 3% BSA/0.1% Tween-20 in PBS and then incubated at room temperature for 1 hour with a mouse monoclonal antibody against denatured cytochrome C (1:500, Pharmingen). An HRP-conjugated goat anti-mouse IgG (1:2000, MBL) was used as a secondary antibody and detected with an ECL (Amersham).

### (7) Detection of Bad protein

Both floating and adhered cardiac myocytes were collected from 75cm² flasks and lysed in 0.5 mL lysis buffer consisting of 10 mM Tris-HCl (pH 7.4), 1 mM EDTA, 1 mM EGTA (pH 8.0), 150 mM NaCl, 1% Triton X-100, 0.2 mM sodium ortho-vanadate, 0.2 mM PMSF and 0.5% NP-40. Bad protein was immunoprecipitated by overnight incubation at 4°C with 5 µl rabbit polyclonal Bad antibody (New England Biolabs). Subsequently, 25 µl protein G-sepharose (Amersham) was added and incubated at 4°C for 1 hour to collect Bad protein. After addition of 25 µl 2X sample buffer, Bad protein was boiled at 95°C for 3 minutes. Each well was loaded with 20 µl sample and electrophoresed on a 15% SDS-polyacrylamide gel. The gel was transferred to a PVDF membrane and probed with a rabbit polyclonal phospho-Bad (Ser136) antibody (New England Biolabs). The total Bad content was determined by probing with a rabbit polyclonal Bad antibody (New England Biolabs). An HRP-conjugated goat anti-rabbit IgG (1:2000, MBL) was used as a secondary antibody and detected with an ECL (Amersham).

### Example 1

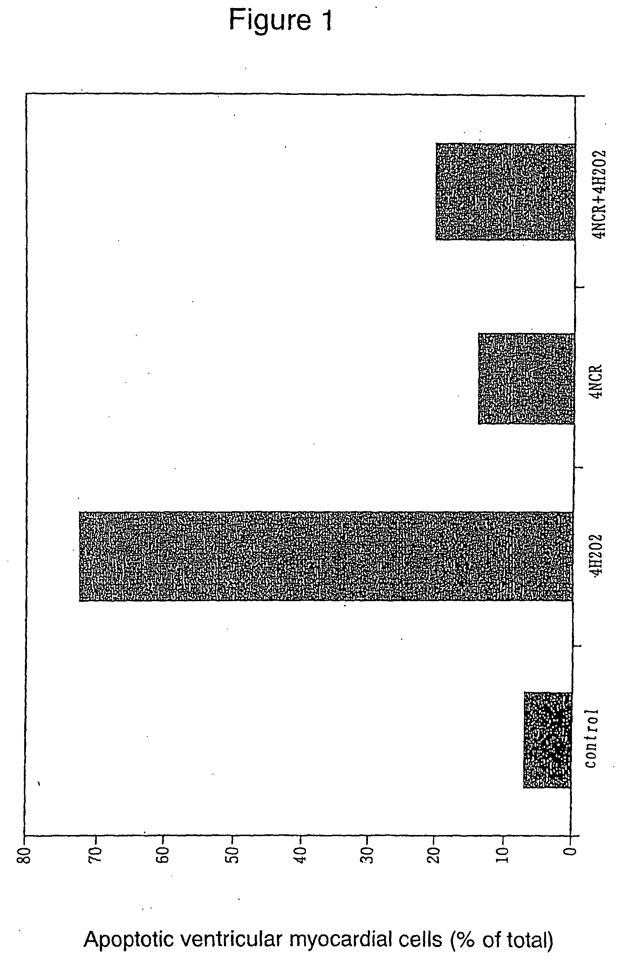

H₂O₂(Sigma) was added at a final concentration of 1 × 10⁻⁴ M to the cell culture of cardiac myocytes prepared as described above (final density: 1 × 10⁶ cells/2 ml/well), followed by culturing for 16 hours in a CO₂ incubator. The cells were collected and analyzed by flow cytometory to determine the percentage of apoptotic cells. Nicorandil was also added at a final concentration of 1 × 10⁻⁴ M immediately before H₂O₂ addition. The results are shown in Figure 1. In Figure 1, "4H2O2" denotes the addition of 1 × 10⁻⁴ M H₂O₂ (final concentration), "4NCR" denotes the addition of 1 × 10⁻⁴ M nicorandil (final concentration), "4NCR+4H2O2" denotes the addition of both 1 × 10⁻⁴ M H₂O₂ (final concentration) and 1 × 10⁻⁴ M nicorandil (final concentration), and "control" denotes a control sample in the absence of all these compounds. The percentage of apoptotic cells was 10% or less in the absence of H₂O₂, whereas apoptosis was induced in more than 70% of the cells in the presence of H₂O₂. On the other hand, H₂O₂-induced apoptosis was significantly inhibited to the control level by addition of 1 × 10⁻⁴ M nicorandil.

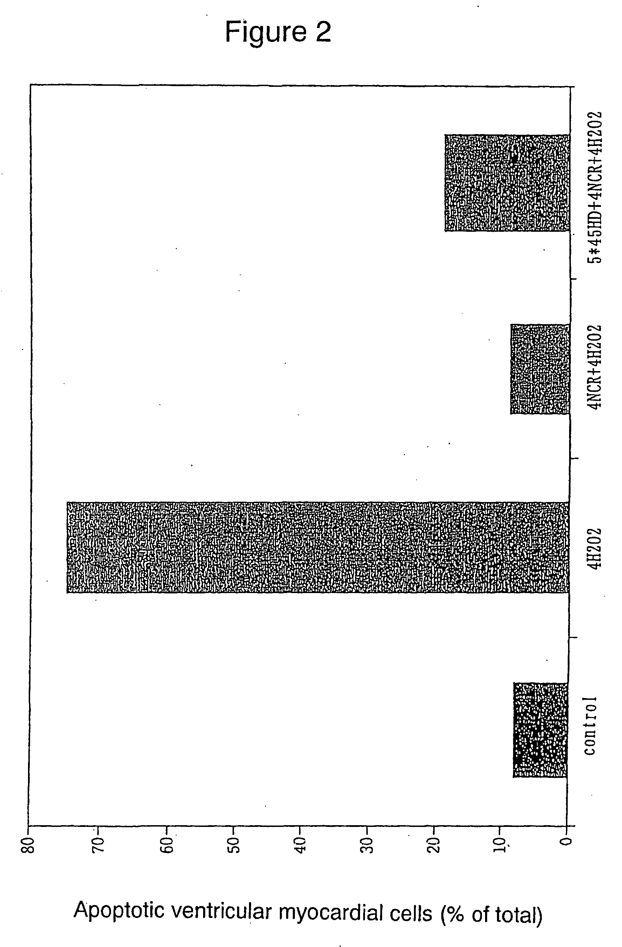

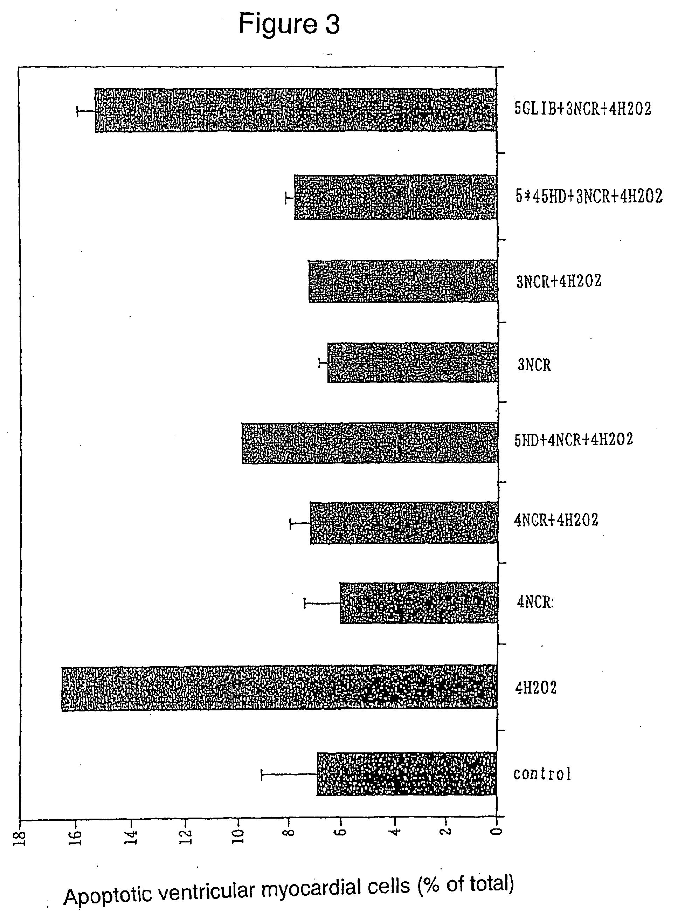

When a mitochondrial K_{ATP} channel inhibitor 5-hydroxydecanoate was further added at a final concentration of 5 × 10⁻⁴ M, the anti-apoptotic effect of nicorandil was partially blocked (Figures 2 and 3). When a non-selective K_{ATP} channel inhibitor glibenclamide was added at a final concentration of 1 × 10⁻⁵ M, the anti-apoptotic effect of nicorandil was completely blocked (Figure 3). In Figure 2, "4H2O2" and "4NCR+4H2O2" are as defined in Figure 1, "5*45HD+4NCR+4H2O2" denotes the addition of 5 × 10⁻⁴ M 5-hydroxydecanoate (final concentration), 1 × 10⁻⁴ M nicorandil (final concentration) and 1 × 10⁻⁴ M H₂O₂ (final concentration), and "control" denotes a control sample in the absence of all these compounds. In Figure 3, "5HD+4NCR+4H2O2" denotes the addition of 5 × 10⁻⁴ M 5-hydroxydecanoate (final concentration), 1 × 10⁻⁴ M nicorandil (final concentration) and 1 × 10⁻⁴ M H₂O₂ (final concentration), "3NCR" denotes the addition of 1 × 10⁻³ M nicorandil (final concentration), "3NCR+4H2O2" denotes the addition of 1 × 10⁻³ M nicorandil (final concentration) and 1 × 10⁻⁴ M H₂O₂ (final concentration), "5GLIB+3NCR+4H2O2" denotes the addition of 5 × 10⁻⁴ M glibenclamide (final concentration), 1 × 10⁻³ M nicorandil (final concentration) and 1 × 10⁻⁴ M H₂O₂ (final concentration). Also, "4H2O2", "4NCR", "4NCR+4H2O2" and "5*45HD+4NCR+4H2O2" are as defined in Figures 1 and 2, and "control" denotes a control sample in the absence of all these compounds.

### Example 2

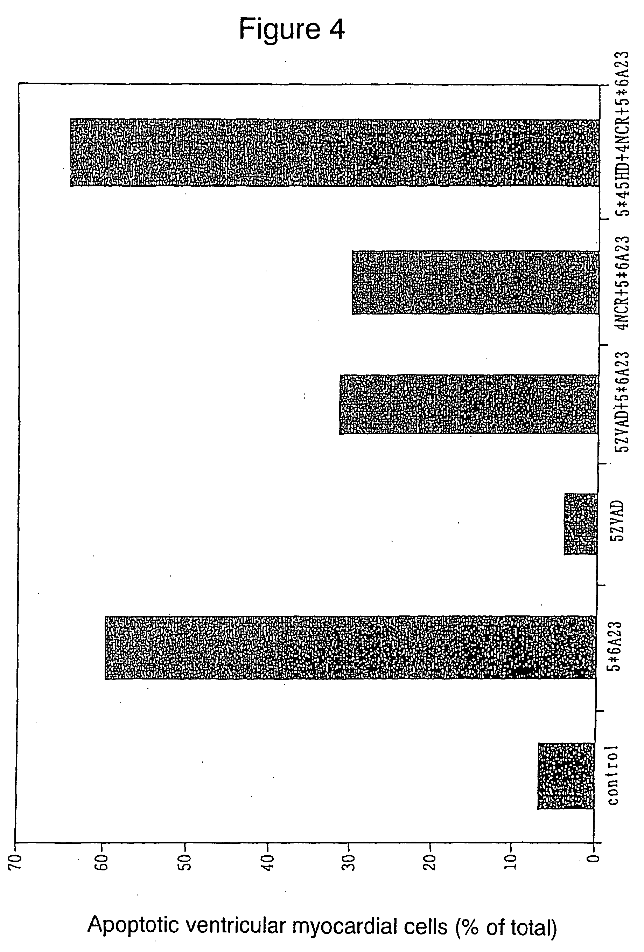

Calcium ionophore A23187 (Sigma) was added at a final concentration of 5 × 10⁻⁶ M to the cell culture of cardiac myocytes prepared as described in Example 1. The cells were cultured as described in Example 1 and analyzed to determine the percentage of apoptotic cells. The results indicated that addition of A23187 induced apoptosis significantly and also confirmed that this pro-apoptosis effect of A23187 was partially (around 50%) blocked by addition of 1 × 10⁻⁵ M ZVAD (pan caspase inhibitor) or 1 × 10⁻⁴ M nicorandil (Figure 4). When a mitochondrial K_{ATP} channel inhibitor 5-hydroxydecanoate was further added at a final concentration of 5 × 10⁻⁴ M, the anti-apoptotic effect of nicorandil was completely blocked (Figure 4). In Figure 4, "5*6A23" denotes the addition of 5 × 10⁻⁶ M calcium ionophore A23187 (final concentration), "5ZVAD" denotes the addition of 1 × 10⁻⁵ M ZVAD (final concentration), "5ZVAD+5*6A23" denotes the addition of 1 × 10⁻⁵ M ZVAD (final concentration) and 5 × 10⁻⁶ M calcium ionophore A23187 (final concentration), "4NCR+5*6A23" denotes the addition of 1 × 10⁻⁴ M nicorandil (final concentration) and 5 × 10⁻⁶ M calcium ionophore A23187 (final concentration), "5*45HD+4NCR+5*6A23" denotes the addition of 5 × 10⁻⁴ M 5-hydroxydecanoate (final concentration), 1 × 10⁻⁴ M nicorandil (final concentration) and 5 × 10⁻⁶ M calcium ionophore A23187 (final concentration), and "control" denotes a control sample in the absence of all these compounds.

Taken together with the results from Example 1, it was indicated that pre-treatment with nicorandil (10⁻⁴ M) produced 60% and 50% inhibition of NRVM apoptosis induced by H₂O₂ and calcium ionophore A23187, respectively.

### Example 3

The apoptotic cells induced in the above Examples were analyzed for DNA fragmentation by ladder assay, confirming that DNA fragmentation was induced in these cells. Likewise, induction of nuclear fragmentation was also confirmed by immunocytostaining (DAPI). These results indicate that myocardial cell death induced by H₂O₂ or A23187 is due to apoptosis. When the apoptotic cells were analyzed for changes in the expression levels of various proteins by Western blot analysis, it was confirmed that there was no change in the protein expression levels of Bcl-2 and Bax, but the protein expression level of p53 was increased. This analysis also confirmed translocation of cytochrome C from mitochondria to cytosol, activation of CPP32, and cleavage of poly(ADP-ribose)polymerase (PARP). These results suggest that a mitochondria-dependent apoptotic pathway was activated to induce DNA fragmentation. Moreover, the results also indicated a decrease in the phosphorylation level of Bad. In contrast, pre-treatment with nicorandil was confirmed to increase the phosphorylation level of Bad. This suggests that the anti-apoptotic effect of nicorandil is mediated by phosphorylation of Bad.

### Example 4

Apoptosis in cardiac myocytes was measured as described in Examples 1 and 2. As a result, the anti-apoptotic effect of nicorandil completely disappeared by addition of a selective PKC inhibitor chelerythrine (final concentration: 10⁻⁶ M). On the other hand, the anti-apoptotic effect of nicorandil was enhanced by addition of an NO donor nitroprusside (final concentration: 10⁻⁴ M), whereas the anti-apoptotic effect of nicorandil was partially blocked by addition of an NOS inhibitor L-NAME (N^{G}-nitro L-arginine methyl ester; final concentration: 10⁻⁶ M). These results suggest that the anti-apoptotic effect of nicorandil in cardiac myocytes is dependent on both a K_{ATP} channel opening effect and a nitrate-like effect, and that nicorandil produces an inhibitory effect on apoptosis through PKC activation via intracellular signaling pathways and Bad phosphorylation.

### Example 5

A fluorescent probe JC-1, a detection reagent for mitochondrial membrane potential, was used to confirm that nicorandil inhibited apoptosis in cardiac myocytes stimulated by H₂O₂. In most cases of cellular apoptosis, cytochrome C is translocated from mitochondrial membrane to cytosol, during which loss of mitochondrial membrane potential is known. Since JC-1 is a cationic fluorescent substance, it easily enters cells and emits red fluorescence (λem = 590 nm) in normal cell mitochondria through formation of polymers called J-aggregates. In contrast, since the mitochondrial membrane potential is disturbed in apoptotic cells, JC-1 cannot be accumulated in the mitochondria and hence exists in the cytosol in a monomer form to emit green fluorescence (λem = 527 nm).

Neonatal rat cardiac myocytes were seeded at a density of 1 × 10⁶ cells/well in 35 mm dishes with glass bottoms (IWAKI) and cultured at 37°C for 10 minutes in serum-free DMEM/F12 medium containing 10 µg/mL JC-1 (Wako Pure Chemical Industries, Ltd.). The cells on the dishes were observed using a confocal laser scanning microscope and a 20× objective lens. Fluorescence was excited by the 488 nm line of an argon laser and the 543 nm line of a helium-neon laser.

The results are shown in Table 5. In the nicorandil-untreated cells shown in the upper panel, most of them were stained in red (spots) before stimulation by H₂O₂ (see upper left), confirming that JC-1 was properly incorporated into the mitochondria. In contrast, after stimulation by H₂O₂ (200 µmol/L, 20 min), the cytosol was diffusely stained in green (upper right; actually observed in yellow from superimposition of the red and green staining), confirming that the mitochondrial membrane potential was disturbed, i.e., apoptosis was induced in these cells. In contrast, pre-treatment with nicorandil (100 µmol/L) was confirmed to inhibit such green fluorescence of cardiac myocytes as produced upon H₂O₂ stimulation (see lower right). These results indicate that pre-treatment with nicorandil inhibits H₂O₂-stimulated apoptosis in cardiac myocytes.

### INDUSTRIAL APPLICABILITY

The present invention is advantageous in providing a clinically effective inhibitor of apoptosis in cardiac myocytes, therapeutic agent for ischemic reperfusion injury, therapeutic agent for myocardial infarction, therapeutic agent for myocardiopathy or therapeutic agent for myocarditis. It is also advantageous in providing a method for screening a compound having at least one of the effects of inhibiting apoptosis in cardiac myocytes, inhibiting. ischemic reperfusion injury, inhibiting myocardial infarction, inhibiting myocardiopathy and inhibiting myocarditis.

## Claims

1. An inhibitor of apoptosis in cardiac myocytes, which comprises a compound having a K_{ATP} channel opening effect and/or an NO donating effect as an active ingredient.

2. The inhibitor of apoptosis in cardiac myocytes according to claim 1, wherein apoptosis in cardiac myocytes is induced by a disease capable of causing calcium overload and/or oxidative stress.

3. The inhibitor of apoptosis in cardiac myocytes according to claim 2, wherein the disease capable of causing calcium overload and/or oxidative stress is ischemic reperfusion injury, myocardial infarction, myocardiopathy or myocarditis.

4. The inhibitor of apoptosis in cardiac myocytes according to any one of claims 1 to 3, wherein the compound having a K_{ATP} channel opening effect and/or an NO donating effect is nicorandil.

5. A therapeutic agent for ischemic reperfusion injury and/or a therapeutic agent for myocardial infarction and/or a therapeutic agent for myocardiopathy and/or a therapeutic agent for myocarditis, each of which comprises a compound having a K_{ATP} channel opening effect and/or an NO donating effect as an active ingredient.

6. The therapeutic agent for ischemic reperfusion injury and/or therapeutic agent for myocardial infarction according to claim 5, wherein the compound having a K_{ATP} channel opening effect and/or an NO donating effect is nicorandil.

7. A therapeutic agent for ischemic reperfusion injury and/or a therapeutic agent for myocardial infarction and/or a therapeutic agent for myocardiopathy and/or a therapeutic agent for myocarditis, each of which comprises a compound having an NO donating effect as an active ingredient, wherein said therapeutic agent is used in combination with an agent having a K_{ATP} channel opening effect.

8. A therapeutic agent for ischemic reperfusion injury and/or a therapeutic agent for myocardial infarction and/or a therapeutic agent for myocardiopathy and/or a therapeutic agent for myocarditis, each of which comprises a compound having a K_{ATP} channel opening effect as an active ingredient, wherein said therapeutic agent is used in combination with an agent having an NO donating effect.

9. A method for inhibiting apoptosis in cardiac myocytes, which comprises exposing cardiac myocytes to a compound having a K_{ATP} channel opening effect and/or an NO donating effect in an amount sufficient to increase the level of phosphorylated Bad in the cardiac myocytes.

10. A method for screening a compound having at least one of the effects of inhibiting apoptosis in cardiac myocytes, inhibiting ischemic reperfusion injury, inhibiting myocardial infarction, inhibiting myocardiopathy and inhibiting myocarditis, which comprises the following steps:
(1) exposing cells to a test compound; and
(2) determining the level of phosphorylated Bad protein present in the cells.

11. An inhibitor of apoptosis in cardiac myocytes and/or a therapeutic agent for ischemic reperfusion injury and/or a therapeutic agent for myocardial infarction and/or a therapeutic agent for myocardiopathy and/or a therapeutic agent for myocarditis, each of which comprises a compound obtained by the method according to claim 10 as an active ingredient.

12. A kit for inhibiting apoptosis in cardiac myocytes, which comprises:
a compound having a K_{ATP} channel opening effect and/or an NO donating effect in an amount effective for inhibiting apoptosis in cardiac myocytes; and
instructions for use.

13. The kit for inhibiting apoptosis in cardiac myocytes according to claim 12, wherein apoptosis in cardiac myocytes is induced by a disease capable of causing calcium overload and/or oxidative stress.

14. The kit for inhibiting apoptosis in cardiac myocytes according to claim 13, wherein the disease capable of causing calcium overload and/or oxidative stress is ischemic reperfusion injury, myocardial infarction, myocardiopathy or myocarditis.

15. The kit for inhibiting apoptosis in cardiac myocytes according to any one of claims 12 to 14, wherein the compound having a K_{ATP} channel opening effect and/or an NO donating effect is nicorandil.

16. A kit for treating ischemic reperfusion injury and/or treating myocardial infarction and/or treating myocardiopathy and/or treating myocarditis, which comprises:
a compound having a K_{ATP} channel opening effect and/or an NO donating effect in an amount effective for treating ischemic reperfusion injury and/or treating myocardial infarction; and
instructions for use.

17. The kit for treating ischemic reperfusion injury and/or treating myocardial infarction and/or treating myocardiopathy and/or treating myocarditis according to claim 16, wherein the compound having a K_{ATP} channel opening effect and/or an NO donating effect is nicorandil.

18. A kit for treating ischemic reperfusion injury and/or treating myocardial infarction and/or treating myocardiopathy and/or treating myocarditis, which comprises:
a compound having an NO donating effect in an amount effective for treating ischemic reperfusion injury and/or treating myocardial infarction and/or treating myocardiopathy and/or treating myocarditis; and
instructions for use,
wherein said kit is used in combination with an agent having a K_{ATP} channel opening effect.

19. A kit for treating ischemic reperfusion injury and/or treating myocardial infarction and/or treating myocardiopathy and/or treating myocarditis, which comprises:
a compound having a K_{ATP} channel opening effect in an amount effective for treating ischemic reperfusion injury and/or treating myocardial infarction and/or treating myocardiopathy and/or treating myocarditis; and
instructions for use,
wherein said kit is used in combination with an agent having an NO donating effect.
